# EUROPEAN PATENT APPLICATION

(11) **EP 4 509 130 A1**
(43) Date of publication of application: **19.02.2025**
(21) Application number: 24157659.4
(22) Date of filing: 14.02.2024
(51) Int. Cl.: A61K 36/185, A61K 31/56

(54) **METHOD FOR PURIFICATION FROM PYRROLIZIDINE ALKALOIDS OF A BIOLOGICALLY ACTIVE PLANT PRODUCT DERIVED FROM TRIBULUS TERRESTRIS CONTAINING FUROSTANOL SAPONINS**

(30) Priority: 14.08.2023 BG 11376223; 07.02.2024 US 202418434887
(71) Applicant: Vemo-99 Ood, 1271 Sofia (BG)
(72) Inventor: Zlatev, Hristo, Sofia (BG); Zlateva, Nina, Sofia (BG); Zlatev, Vasil, Sofia (BG); Kirkov, Lubomir, Sofia (BG)
(74) Representative: Tarpanova, Antoaneta

(57) **Abstract**

The invention relates to a composition of matter extracted from the plant Tribulus terrestris, having less than 400 µg per kilogram of dry matter pyrrolizidine alkaloids and at least 40% furostanol saponins and a Tribulus terrestris extract having at least 10% furostanol saponins and a concentration of pyrrolizidine alkaloids of less than 80 µg per liter extract. The invention is further directed to a method for the purification of pyrrolizidine alkaloids from Tribulus terrestris plant extract by salting out the saponins from the extract with a first salt solution until a precipitate forms, washing the precipitate with a second salt solution and then dissolving the precipitate in water, removing the remaining amount of salt from the obtained aqueous solution by extraction with alcohol, washing with water, and evaporating the residual alcohol to a final Tribulus terrestris extract, which can be dried.

## Description

### FIELD OF THE INVENTION

The invention relates to a method for the purification of biologically active plant products containing furostanol saponins by reduction of the content of pyrrolizidine alkaloids, and to a biologically active plant product, derived from Tribulus terrestris, containing furostanol saponins and reduced amount of pyrolizidine alkaloids, which are used in the pharmaceutical and supplement industry.

### TECHNICAL BACKGROUND

There are well-known methods for obtaining biologically active products from various plant raw materials, which involve extraction with water or water-alcohol mixtures. (Abubakaret. Al.,J PharmBioalliedSci. 2020 Jan-Mar; 12(1):1-10; Simeonov, E. et al. Journal of the University of Chemical Technology and Metallurgy, 46, 3, 2011).

The products obtained by the extraction are total herbal extracts containing an uncontrollable amount of active ingredients, undesirable impurities and ballast substances. In order to become suitable for use in accordance with the physiological targeting of their active ingredients and to meet the requirements for a reduced content of accompanying harmful substances, the total extracts need to be subjected to series of purifications. These purifications remove interfering substances and concentrate the active substances in the extract.

Different methods have been developed for the extraction of alkaloids and in particular regarding the pyrrolizidine ones. (Thomas Kopp et al., Toxins 2020, 12, 320;; Al-Subaie et al.,. Foods 2022, 11, 3873; Tosun et al., FABAD J. Pharm. Sci., 19, 149-151, 1994; M. Willocx et al., Food Chemistry: Molecular Sciences 4 (2022) 100070;

Autors' collective. Chemical analysis of medicinal plants: tudy, manual for pharmaceutical universities -§3. Extraction methods/ Ladygina E. Ya and others. In order. Grinkevich N.I., Safronich .N.-M.: University 1983, -176 p., III. 1983: Methods of extraction of alkaloids from plant raw materials, Medicinal substances belonging to the group of alkaloids. Derivatives quinolysine, pyrrolizidine, piperidine, tropane, quinoline, isoquinoline. Pharmaceutical chemistry lecture #1-2, Methds of extracting alkaloids form plant raw materials (studopedia.su); Chemistry and technology of chemical pharmaceutical preparations - GENERAL METHODS EXRACTION OF ALKALOIDS FORM PLANTS.[c.523];)

Pyrrolizidine alkaloids are usually extracted from a dried, ground plant material with the help of a hot or cold alcohol. The alcohol is evaporated, the remaining bases are neutralized with a diluted acid, and the fats are extracted with ether or petroleum. Ordinarily at this stage, all the present N-oxides are reduced with zinc and are transformed to the corresponding basic alkaloids, before making the solution alkaline and extracting the alkaloids with chloroform (Koekemoer & Warren, 1951).

As an alternative, the alcohol may circulate continuously through the plant material and then circulate through a cation exchange resin, and subsequently the alkaloids could be eluted from the resin (Mattocks, 1961; Deagen & Deinzer, L977).The pyrrolizidine alkaloids can also be extracted by soaking the plant material in a diluted aqueous solution of an acid (Briggsetal., 1965,' Craigetal., 1984).

There are different known methods for extracting biologically active substances from the Tribulus terrestris. (C. Tian et al, Heliyon 5 (2019) e02234; V. Sasikala et al., International Journal of Engineering Research & Technology (IJERT) ISSN: 2278-0181 Vol. 7 Issue 12, December-2018).

One such method, for instance, is described in BG 65737 B1, where the extraction from the ground drug, obtained from the aerial part of Tribulus terrestris is performed at a temperature from 50 to 55°C, with a water-methanol solvent, where the ratio water-methanol vary from 1:1 to 1:99. The extract is treated with activated carbon and, after filtration, it is concentrated under vacuum until the complete removal of the methanol. The resulting aqueous concentrate is extracted with methylene chloride. This is followed by a distillation of the methylene chloride, which is thus regenerated and used again in the proceeding. The purified aqueous extract is acidified with hydrochloric acid to pH 3-5 and then extracted several times with water-saturated n-butanol. The butanol extracts thus obtained are purified in two ways. According to the first method, the butanol extracts are washed with sodium chloride solution and concentrated under vacuum to a dry residue, which is dissolved in a lower alcohol. The resulting solution is added drop-by-drop to acetone and left to sit for about 24 hours at 1 0-15°C, then it is filtered, washed with acetone and dried.

According to the second method, the butanol extracts are washed with a 0.5-1.5% sodium hydroxide solution. After separation of the two layers, the butanol layer is washed with water to pH 7, concentrated under vacuum until the butanol is completely removed, and the residue is dried.

It is known, for example, that the plant Tribulus terrestris contains protodioscin. Protodioscin is a type of saponin having a steroid structure, which is used in the treatment of urinary infections, colic, and which has a general tonic effect and stimulates the reproductive system functions. The Tribulus terrestris plant extract also contains other accompanying active substances that modulate and enhance the protodioscin effects. (Mengquan Li et al. Cell PhysiolBiochem 2013; 32:1299-1308).

The other furostanol saponins present in the extract play an important role in the main properties of protodioscin to be manifested. They help to transform the cholesterol into dehydroepiandrosterone (DHEA). DHEA is thought to slow down the aging process by participating in the normal steroid synthesis in the body, helping with the transformation of free cholesterol into sex hormones. The reduced cholesterol levels indirectly maintain the effects of the extract's active substances on the cholesterol metabolism. According to some scientists, the plant extract stimulates the synthesis of luteinizing hormone and naturally increases the testosterone levels. The testosterone boosts the growth of muscle mass indirectly by stimulating the synthesis of protein in the body.

The benefits of using the extract of Tribulus terrestris are well known: the extract helps reduce blood sugar, improves blood circulation, helps the maintenance of the cardiovascular and liver functions, and stimulates the immune system. (Zhu et al. Chemistry Central Journal (2017) 11:60).

The Tribulus terrestris extract also has a tonic effect, and it helps to overcome physical and mental overworks. It is suitable to be included in various nutritional supplements, designed to increase strength, muscle growth and lean muscle mass. The extract is designed to support athletes in the preparatory, active and recovery periods, after physical efforts or after a recent illness. It helps to stimulate human sexuality and libido, positively affects the functions of the prostate gland and the general physiological state of the body, and supports the normal state of the female organism during the menopause.The prolonged use does not lead to a sensibility, dependence or addiction.

The pyrrolizidine alkaloids are some of the undesirable substances that are extracted along with the furostanol saponins. They can either be naturally present in the herb or can be the result of the presence of unwanted botanical herbal impurities. According to the studies, frequent and large consumption of honey, tea, herbal infusions and food supplements with high content of pyrrolizidine alkaloids could have harmful effect on human health, as it is considered that pyrrolizidine alkaloids in large amounts could have a genotoxic carcinogenic impact on humans. (Environmental Health Criteria 80, Pyrrolizidine alkaloids, WHO, Geneva, 1988).

The known methods for extraction of pyrrolizidine alkaloids are not suitable for the extraction from Tribulus terrestris, because their behavior is similar to that of the furostanol saponins, which leads to the significantly low concentration of the latter in the extract. During the extraction and the subsequent purifications of the Tribulus terrestris extract, the pyrrolizidine alkaloids cannot be separated from the furostanol saponins, as the latter are removed from the extract along with them.

The series of experiments conducted with a changing pH, through the addition of sodium carbonate (Na2CO3), citric acid, ammonia, etc. during the extraction and prior to the subsequent purifications with butanol, establish that despite the purification of a large portion of the pyrrolizidine alkaloids from the extract, the levels of the pyrrolizidine alkaloids in the obtained purified product remain close to 2,000 µg/kg. The attempts for the purification of the extract from pyrrolizidine alkaloids with acetone led to a decrease in the yield by 50%.

### DETAILED DESCRIPTION

The goal of the invention is to create a method for the purification of biologically active plant products, derived from of Tribulus terrestris extracts and containing furostanol saponins, from pyrrolizidine alkaloids.

The purpose of the invention is to provide a biologically active product derived from Tribulus terrestris with a low content of alkaloids and a high content of furostanol saponins.

The invention is directed to a composition of matter extracted from the plant Tribulus terrestris having at least 40% furostanol saponins and pyrrolizidine alkaloids, having concentration of less than 400 µg per kilogram of said composition. The invention is directed also to a Tribulus terrestris extract comprising at least 10% furostanol saponins and pyrilizidine alkaloids, having concentration of less than 80 µg per liter extract.

The goal is achieved by using a method for the reduction of pyrrilizidine alkalioids content in Tribulus terrestris extract, comprising furostanol saponins.

The invention is further directed to a method for reducing the content of pyrrolizidine alkaloids to below 80 µg per liter in a Tribulus terrestris extract, which contains furostanol saponins. The method includes the steps of salting out the furostanol saponins by adding a first salt solution to an initial Tribulus terrestris extract until a precipitate forms, collecting and washing the precipitate with a second salt solution, collecting the washed precipitate and dissolving the washed precipitate in water to obtain an aqueous extract, performing alcohol extraction by adding immiscible in water alcohol to the aqueous extract to obtain an aqueous phase and an alcohol phase, thereafter discarding the aqueous phase, collecting the alcohol phase and washing the alcohol phase with water, finally evaporating the alcohol from the washed alcohol phase (alcohol extract) until a final Tribulus terrestris extract is obtained.

Optionally, the final Tribulus terrestris extract can be dried. Preferably, the drying is carried out in a spray dryer at an input temperature of 95-85°C and an output temperature of 195-185°C.

The first salt solution added to the initial Tribulus terrestris extract for the salting out of the furostanol saponins is preferably a solution of sodium chloride or potassium chloride. Preferably, the second salt solution used for the washing of the precipitate, resulting from the salting out of the furostanol saponins is a solution of sodium chloride or potassium chloride. The immiscible alcohol is an alcohol, which when mixed with water at room temperature results in phase separation. Immiscble alcohols are n-butanol and isobutanol, pentanol, hexanol or a combination thereof. Preferably, the alcohol used for the alcohol extraction is n-butanol.

In an embodiment, the ratio of the salt solution for the salting out of the furostanol saponins step is from 1:2 to 1:8, initial Tribulus terrestris extract to first salt solution, the ratio of the second salt solution for the washing of the resulting precipitate step is from 1:0.5 to 1:2, precipitate to second salt solution. Preferably, the washing is repeated 2 to 6 times. The volume ratio of the washed precipitate dissolved in water is preferably from 1:2 to 1:4, washed precipitate to water, the volume ratio of aqueous extract to alcohol in alcohol extraction step is from 1:2 to 1:6 aqueous extract to alcohol, and the ratio of the water for the washing of the alcohol phase step is from 1:2 to 1:6 water to alcohol phase.

In a preferred embodiment, the initial Tribulus terrestris extract is an aqueous extract.

In a preferred embodiment, the ratio of the first salt solution for the salting out of the furostanol saponin step is 1:4, initial Tribulus terrestris aqueous extract to salt solution.

In a preferred embodiment, the ratio of the precipitate to salt solution for the washing of the precipitate step is 1:1. The washing of the precipitate step is repeated preferably five times.

In a preferred embodiment, the volume ratio of the washed precipitate to water in the dissolution of the precipitate in water step is from 1:3, washed precipitate to water.

In a preferred embodiment, the volume ratio of the immiscible in water alcohol in the alcohol extraction step is 1:4 water to alcohol.

Prefereably, the water used for the washing of the alcohol phase is in ratio 1:4 water to alcohol phase.

The initial aqueous extract, according to an embodiment of the invention, is obtained by a method including the following steps. First, extracting Tribulus terrestris from ground Tribulus terrestris herb with 20% to 80% lower alcohol, (lower alcohol is methanol, ethanol or propanol, or a combination thereof) in ratio from 1:2 to 1:6 kilogram of herb to liters of alcohol, under stirring conditions, at a temperature of 30°C to 60°C for 2 to 8 hours, and repeating the extraction with lower alcohol at least three times and collecting the extracts, obtaining a first aqueous concentrated by concentrating the extracts until the alcohol is completely removed. Second, extracting the Tribulus terrestris from the first aqueous concentrate with aqueous butanol in ratio of 1:2 to 1:6 aqueous concentrate to aqueous butanol, at a temperature of 20°C to 40°C until a butanol phase and an aqueous phase form, repeating the extraction with aqueous butanol at least four times and collecting the butanol phases, then removing the butanol from the butanol phases until a second aqueous concentrate is obtained. Third, subjecting the aqueous concentrate to extraction with chloroform by adding chloroform to the second aqueous concentrate in volume ratio from 1:1 to 1:4 aqueous concentrate to chloroform at a temperature of 20°C to 30°C until aqueous phase and chloroform phase form, repeating the extraction with chloroform at least three times, collecting the aqueous phases obtained after each extraction and removing the remaining chloroform from the aqueous phases until a concentrated aqueous extract of Tribulus terrestris is obtained.

Another embodiment of the invention is a Tribulus terrestris extract, obtained by the disclosed method for reducing the content of pyrrolizidine alkaloids and said extract containing at least 10% furostanol saponins and pyrrolizidine alkaloids having concentration of less than 80 µg per liter of said extract.

A different embodiment of the invention is a composition of matter extracted from the plant Tribulus terrestris and subjected to the disclosed method for reducing the content of pyrrolizidine alkaloids, where said composition contains at least 40% furostanol saponins and pyrrolizidine alkaloids, having concentration of less than 400 µg per kilogram of said composition.

The goal of reducing the content of pyrrolizidine alkaloids in Tribulus terrestris extract, containing furostanol saponins is more specifically achieved by using a method with the following characteristics: first, the furostanol saponins in the biologically active plant product obtained by extraction are salted out by adding a saturated solution of sodium chloride or potassium chloride to the aqueous concentrate of the extract of the biologically active plant product until a precipitate forms. The so-formed precipitate is washed several times with a saturated solution of sodium chloride or potassium chloride. Then, the washed precipitate is dissolved in distilled water to obtain an aqueous extract. The residual amount of sodium chloride or potassium chloride is separated from the aquous extract by consecutive extractions with butanol. After each extraction the butanol is washed with water. Finally, the butanol is evaporated and a pure aqueous concentrate of the biologically active plant product is obtained.

According to one embodiment of the invention, the furostanol saponins of the extracted biologically active plant product are salted out by adding a saturated salt solution of sodium chloride or potassium chloride to the concentrated aqueous extract of the biologically active plant product in a ratio of 1:2 to 1:8, concentrated aqueous extract of the biologically active plant product to the saturated salt solution, until a precipitate is formed. The precipitate obtained is subjected to 2 to 6 washings with a saturated solution of sodium chloride or potassium chloride in a ratio of 1:0.5 to 1:2, precipitate to the saturated salt solution of sodium or potassium chloride, until the content of pyrrolizidine alkaloids decreases below 80 µg/l of concentrate. The resulting purified precipitate is dissolved in distilled water in a volume ratio of 1:2 to 1:4, precipitate to water, to obtain an aqueous extract. The residual amount of sodium chloride or potassium chloride is removed from the resulting aqueous extract by successive extractions with butanol in volume ratio from 1:2 to 1:6 water extract to butanol. After each extraction, the butanol is washed with water in a ratio of 1:2 to 1:6 water to butanol. The butanol is evaporated to obtain a pure aqueous concentrate of the biologically active plant product.

The recommended ratio between the concentrated aqueous extract of the biologically active plant product to the saturated solution of sodium chloride or potassium chloride used in the salting-out process of furstanol saponins is 1:4.

It is recommended that the washings of the precipitate obtained from salting out with a saturated solution of sodium chloride or potassium chloride be performed with saturated solution in ratio 1:1 precipitate to saturated solution.

It is further recommended that the furostanol saponin precipitate be subjected to 5 washings with saturated sodium chloride or potassium chloride solution.

The optimal ratio for the dissolution of the purified precpitate, is 1:3 purified precipitate to distilled water.

It is recommended that three successive extractions with butanol at a ratio of 1:4 aqueous extract to butanol be performed.

It is advisable that the washings after each butanol extraction be performed with water in a ratio of 1:4 water to butanol.

According to another version of the invention, the biologically active plant product, derived from Tribulus terrestris, is obtained by successive extractions with lower alcohol, aqueous butanol and chloroform, as follows: the ground drug Tribulus terrestris, undergoes at least three extractions with 20% to 80% lower alcohol in a ratio of 1:2 to 1:6 kilogram of herb to liters of alcohol) under shaking conditions, at a temperature of 30°C to 60°C for 2 to 8 hours. After each extraction, the extracts are separated. Then, the extracts obtained from the lower alcohol extractions are combined and concentrated until the alcohol is completely removed and an aqueous concentrate is obtained. The resulting aqueous concentrate is subjected to at least four successive extractions with aqueous butanol, adding the aqueous butanol to the aqueous concentrate in a volume ratio of 1:2 to 1:6 aqueous concentrate to aqueous butanol, with a waiting time of 2 to 8 hours for the phases to separate at a temperature of 20°C to 40°C. The butanol phases of each extraction are collected after separation. They are combined and concentrated until the butanol is completely removed and an aqueous concentrate is obtained. The aqueous concentrate obtained after the butanol extraction undergoes at least three successive extractions with chloroform in a volume ratio of 1:1 to 1:4 aqueous concentrate to chloroform, left for 2 to 8 hours at a temperature of 20°C to 30°C. Each chloroform extraction forms an aqueous phase and a chloroform phase. The chloroform phases are removed, and the resulting aqueous phase is boiled until the chloroform is completely removed. Thus, a concentrated aqueous extract of the plant's biologically active product is obtained.

In order to achieve a pure dry extract, the resulting pure aqueous concentrate, purified by this purification method, is dried. It is recommended to dry the resulting pure water concentrate in a spray dryer at an input temperature of 95-85°C and an output temperature of 195-185°C until a pure dry extract is obtained.

According to the invention, an aqueous extract of a biologically active product on the basis of Tribulus terrestris, purified from pyrrolizidine alkaloids, is obtained. The product contains 10-20% furostanol saponins and pyrrolizidine alkaloids, having concentration of less than 80 µg per liter of aqueous concentrated solution.

According to the invention, a dry biologically active plant product derived from Tribulus terrestris, purified from pyrrolizidine alkaloids, is created. This dry product contains 40-75% furostanol saponins and pyrrolizidine alkaloids, having concentration of less than 400 µg per kilogram of dry product.

One of the advantages of this invention is that the present purification method can be applied to the purification of other biologically active plant products containing furostanol saponins from pyrrolizidine alkaloids. Also, the method is applicable to products extracted from the Tribulus terrestris plant using different extraction ways.

When using the invention, a purified biologically active product derived from Tribulus terrestris is obtained. The purified aqueous extract has a low content of pyrrolizidine alkaloids - less than 80µg/l concentrate, with a relatively high concentration of furostanol saponins - 10-20%, and the purified dry extract has less than 400µg pyrrolizidine alkaloids per kilogram of dry product and a content of 40-75% furostanol saponins. Thus, the beneficial effects of the product are preserved, while the harmful ingredients are reduced.

The following examples are provided for illustrative purposes only, and the scope of the present invention shall not be limited thereto in any way.

### Examples of the invention's implementation

### Preparation of Tribulus Terrestris extract purified from pyrrolizidine alkaloids

### Example 1

Four liters of 60% isopropyl alcohol are added to 1 kg of the dry ground drug Tribulus terrestris. The extraction is carried out with moderate stirring of the resulting mixture for 4 hours at a temperature of 30°C. The extract is separated. The once extracted drug, is re-extracted with isopropyl alcohol in a ratio of 1:4 - 4 liters of 60% isopropyl alcohol per 1 kg of the herb, used in the first extraction, at 30°C for four hours with moderate stirring. The extract is separated, and the drug undergoes a third extraction under the same conditions. The extracts obtained from the three extractions are combined and concentrated at 0.9 MPa vacuum and a temperature of 50°C until the isopropyl alcohol is completely removed and a first aqueous concentrate is obtained.

The extraction is performed with a lower alcohol such as methanol, ethanol or isopropanol, preferably isopropanol.

The resulting aqueous concentrate undergoes four extractions with aqueous n-butanol. The aqueous n-butanol is used for purification purposes, in order to achieve an optimal extraction of the furostanol saponins. The first extraction is carried out by adding aqueous n-butanol to the previously obtained aqueous concentrate in a volume ratio of 1:4, extract to aqueous n-butanol. It takes 4-5 hours for the separation of the phases. The separation takes place at 20°C. Thereafter, the aqueous phase is removed.

For the second extraction, aqueous n-butanol is added to the n-butanol phase from the first extraction, in a ratio of 1:4, n-butanol phase to aqueous n-butanol, and is left to sit for 4-5 hours at 20°C until the complete separation of the phases. For the third and fourth extractions, an aqueous n-butanol is added again to the n-butanol phase that had passed through the previous extractions, respectively through the second and the third, in a ratio of 1:4, n-butanol phase to aqueous n-butanol, and is left to sit for 4-5 hours at 20°C until the phases separate. The aqueous phase is removed after each extraction.

The n-butanol phases are combined and concentrated, the concentration being carried out under 0.9 MPa vacuum and at a temperature of 50°C until a second aqueous concentrate is obtained and the n-butanol is completely removed.

The resulting second aqueous concentrate after the extractions with aqueous n-butanol undergoes three extractions with chloroform in a volume ratio of 1:2, concentrate to chloroform, where it is left for 4 hours at 20°C for the phase separation. The chloroform phase after each extraction is removed.

The aqueous phase obtained after the three chloroform extractions is boiled until the chloroform is completely removed.

The content of furostanol saponins in the resulting aqueous extract before undergoing purifications is 15%, respectively, about 62% calculated on dry matter. The content of furostanol saponins in the obtained aqueous extract is determined with the help of the UV-VIS spectrophotometric method, comparing the tested solution with a control standard solution of the protodioscin substance at a wavelength of 515 nm.

The aqueous extract of Tribulus terrestris, which is obtained according to the described steps is subsequently purified according to the present invention. The saponins are salted out by adding a saturated solution of sodium chloride or potassium chloride to the concentrated aqueous extract, in a ratio of 1:4, concentrated aqueous extract to a saturated salt solution, whereby a precipitate of furostanol saponins is formed. The precipitate obtained is washed 5 times with a saturated solution of sodium chloride or potassium chloride, in a ratio of 1:1, precipitate to salt solution, after which the precipitate already purified from pyrrolizidine alkaloids is dissolved in distilled water in a volumetric ratio of 1:3, precipitate to water.

The dissolved precipitate (aqueous extract) undergoes three extractions with n-butanol in a volume ratio of 1:4, dissolved precipitate (aqueous extract) to n-butanol, for the complete removal of the salt: sodium chloride or potassium chloride. After each n-butanol extraction, the used n-butanol is washed with water at a ratio of 1:4 (water to butanol). The n-butanol phases are combined and evaporated under a 0.9 MPa vacuum at a temperature of 50°C and 300 ml of pure aqueous concentrate is obtained. The content of the furostanol saponins in thus obtained pure aqueous concentrate is 15.2%, determined by the UV-VIS spectrophotometric method, where the test solution is compared to control standard solutions of the substance protodioscin at a wavelength of 515 nm.

The pure water concentrate is dried in a spray dryer at an input temperature of 95 - 85 °C and an output temperature of 195 - 185 °C until a clean dry product is obtained. The content of the furostanol saponins in the dry product is 62.4%, and the concentration of pyrrolizidine alkaloids is 64.3 µg/kg. The concentration of pyrrolizidine alkaloids is determined by the LC-MS/MS method.

### Example 2

Four liters of 40% isopropyl alcohol are added to 1 kg of the dry, ground Tribulus terrestris drug. The extraction is carried out by moderately stirring the resulting mixture for 4 hours at a temperature of 40°C. The extract is separated. The drug, already extracted once, is re-extracted with isopropyl alcohol in a ratio of 1:4 - 4 liters of 40% isopropyl alcohol per 1 kg of herb from the first extraction, at 40°C for four hours with moderate stirring. The extract is separated and the drug is submitted to a third extraction under the same conditions. The extracts, that are obtained from the three extractions are combined and concentrated under vacuum of a 0.9 MPa, at a temperature of 60°C, until the complete removal of the isopropyl alcohol is achieved and an aqueous concentrate is obtained.

The resulting aqueous concentrate undergoes four extractions with aqueous butanol.

For the optimal extraction of the furostanol saponins, an aqueous butanol is used for the purification.

The first extraction is carried out by adding aqueous butanol to the obtained aqueous concentrate in a volume ratio of 1:4, extract to aqueous butanol. It takes 4-5 hours for the phases to separate. The separation takes place at 20°C, after which the aqueous phase is discarded.

The second extraction is carried out with the addition of aqueous butanol to the butanol phase, which has passed through the first extraction in ratio of 1:4, and is left to sit at 20°C for 4-5 hours again, until the separation of the two phases.

In the third and fourth extractions, aqueous butanol is added again to the butanol phase that passed through the previous extraction, respectively the second and the third extractions in a ratio of 1:4 butanol phase to aqueous butanol. Again, the mixture is left to sit at 20°C for 4-5 hours until the phases separate. Thereafter the aqueous phase is separated. The butanol phases are combined and concentrated under a 0.9 MPa vacuum at a temperature of 50°C until the butanol is completely removed and an aqueous concentrate is obtained.

The concentrate, which was obtained from the extractions with aqueous butanol undergo three extractions with chloroform in a volume ratio of 1:2, extract to chloroform. The separation of the phases is completed after 4 hours at 20°C. After each extraction the chloroform phase is discarded.

The aqueous concentrate obtained after the three chloroform extractions is boiled at atmospheric pressure until the chloroform therefrom is removed completely.

The content of furostanol saponins in the thus obtained aqueous extract prior to undergoing any purification is 10%, respectively 45%, calculated derived from dry matter. The content of furostanol saponins in the obtained aqueous extract is measured by the UV-VIS spectrophotometric method, which compares the tested solution with a standard control solution of the substance protodioscin at a wavelength of 515 nm. The aqueous extract is concentrated under 0.9 MPa vacuum at a temperature of 50°C to a concentrate of 40% dry matter.

The resulting concentrated aqueous extract of Tribulus terrestris, is purified from pyrrolizidine alkaloids according to the method described below.

The furostanol saponins are salted out by adding a saturated salt solution - sodium chloride or potassium chloride, to the concentrated aqueous extract, in a ratio of 1:4 (concentrated aqueous extract to saturated saline solution), which results in a precipitate of furostanol saponins. This precipitate is washed 5 times with a saturated solution of sodium chloride or potassium chloride in a ratio of 1:1, precipitate to saline solution, after which the precipitate already purified from pyrrolizidine alkaloids is dissolved in distilled water in a volumetric ratio of 1:3, precipitate to water.

For the complete removal of the sodium chloride or potassium chloride, the dissolved precipitate undergoes three extractions with butanol at a volume ratio of 1:4, dissolved precipitate to butanol. After each butanol extraction, the used butanol is washed with water at a ratio of 1:4, water to butanol.

The residual butanol is evaporated under 0.9 MPa vacuum at a temperature of 50°C to obtain 350 ml of pure aqueous concentrate. The content of furostanol saponins in the pure aqueous concentrate is 10.5%. The pure aqueous concentrate, thus obtained, is dried in a spray dryer at an input temperature of 95-85 °C and an output temperature of 195-185 °C. The content of furostanol saponins in the dry product, determined through the LC-MS/MS method, is 45.6%, while the concentration of pyrrolizidine alkaloids is evaluated at 115.1 µg/kg.

Table 1 represents the content of pyrrolizidine alkaloids in the intermediate and final products obtained with and without purification.

**Table 1**

| Product | Description | Content of pyrrolizidine alkaloids µg/kg | Content furostanol saponins, % |
|---|---|---|---|
| Extract of Tribulus terrestri s | Dry extract, extracted with 60% isopropanol (IPA) at 30°C followed by butanol and chloroform purifications | 49 700 | 69.9 |
| | Dry extract, extracted with 20% IPA at 30°C | 27 600 | 6.0 |
| | Dry extract, extracted with water at 70°C | 8 800 | 7.6 |
| | Dry extract, extracted with 60% IPA at 30°C | 17 700 | 11.2 |
| | Analysis of the final products from various batches that were purified by reduction of the pyrrolizidine alkaloids content. | | |
| | Dry extract, extracted with 60% isopropanol (IPA) at 30°C followed by butanol and chloroform purifications | 45 | 58.2 |
| | Dry extract, extracted with 60% isopropanol (IPA) at 30°C followed by n-butanol and chloroform purifications | 64.3 | 62.4 |
| | Dry extract, extracted with 40% isopropanol (IPA) at 40°C followed by butanol and chloroform purifications | 115,1 | 45.6 |

Table 2 represents data of the content of furostanol saponins and pyrrolizidine alkaloids in Tribulus terrestris' dry extracts, obtained by using different extraction methods

**Table 2**

| Pyrrolizidine alkaloids content in Tribulus Terrestris extract, obtained according to the present invention | | Pyrrolizidine alkaloids content in Tribulus terrestris extract obtained by using another standard method | |
|---|---|---|---|
| Extraction with 60% IPA, purifications with n-butanol | 62,4 % (furostanol saponins), | Changing the pH of the herb with Na₂CO₃ at the isopropanol extraction; | 69.2 % ((furostanol saponins), 34 500µg/kg |
| and chloroform, precipitation with saturated NaCl, washes with saturated NaCl, n-butanol purifications from NaCl | 64,3 µg/kg (pyrrolizidine alkaloids) | Centrifugation; Chloroform purification; Correction of the pH 3.6 with citric acid before the butanol purification. | (pyrrolizidine alkaloids) |
| Extraction with 40% IPA, purifications with butanol and chloroform,, precipitation with saturated NaCl, washes with saturated NaCl, butanol purifications from NaCl | 45,6 % (furostanol saponins), | Changing the pH of the herb with Na₂CO₃ during the isopropanol extraction; Correction of the pH 4.6 with citric acid before centrifugation; Chloroform purification; Correction of the pH 3.6 with citric acid before the butanol purification. | 76.5 % (furostanol saponins), |
| | | | 21 200 µg/kg (pyrrolizidine alkaloids) |
| | 115,1 µg/kg (pyrrolizidine alkaloids) | | |

The biologically active plant based product obtained according to the invention is a Tirbulus terrestris plant's extract, which has the advantage of having 10-20% furostanol saponins and no more than 80 µg of pyrrolizidine alkaloids per liter extract, before drying, and in its dry form it contains 40-75% furostanol saponins and less than 400µg of pyrrolizidine alkaloids per kilogram dry product.

The present method can also be applied to the purification of extracts from other plants containing furostanol saponins and pyrrolizidine alkaloids. In order for this method to work effectively, the saponins need to be purified from other interfering impurities such as e.g. chlorophyll.

## Claims

1. A composition of matter extracted from the plant Tribulus terrestris comprising at least 40% furostanol saponins and **characterized in that** said composition has pyrrolizidine alkaloids of less than 400 µg per kilogram of said composition.

2. A Tribulus terrestris extract comprising at least 10% furostanol saponins and **characterized in that** said extract has pyrilizidine alkaloids concentration of less than 80 µg per liter extract.

3. A method for reducing the content of pyrrolizidine alkaloids to below 80 µg per liter in a Tribulus terrestris extract comprising furostanol saponins, said method comprising the following steps:
a. salting out the furostanol saponins by adding a first salt solution to an initial Tribulus terrestris extract until a precipitate forms;
b. collecting and washing the precipitate with a second salt solution;
c. collecting the washed precipitate and dissolving the washed precipitate in water to obtain an aqueous extract;
d. adding immiscible in water alcohol to the aqueous extract to form aqueous phase and alcohol phase;
e. discarding the aqueous phase and washing the alcohol phase with water;
f. evaporating the alcohol from the washed alcohol phase to obtain a final Tribulus terrestris extract having pyrrolizidine alkaloids concentration of below 80 µg per liter.

4. The method according claim 3, wherein:
a. the first salt solution for the salting out of the furostanol saponins in step a is a solution of sodium chloride or potassium chloride;
b. the second salt solution for the washing of the precipitate in step b is a solution of sodium chloride or potassium chloride;
c. the alcohol for the alcohol extraction in step d is n-butanol.

5. The method according to claim 3, wherein the initial Tribulus terrestris extract is an aqueous extract.

6. The method according to claim 5, wherein:
a. the ratio of the salt solution for the salting out of the furostanol saponins in step a is from 1:2 to 1:8, initial Tribulus terrestris extract to first salt solution;
b. the ratio of the second salt solution for the washing of the resulting precipitate in step b is from 1:0.5 to 1:2, precipitate to second salt solution, and the washing is repeated 2 to 6 times;
c. the volume ratio of the washed precipitate dissolved in water in step c is from 1:2 to 1:4, washed precipitate to water;
d. the volume ratio of aqueous extract to alcohol in step d is from 1:2 to 1:6 aqueous extract to alcohol;
e. the ratio of the water for the washing of the alcohol phase in step e is from 1:2 to 1:6 water to alcohol phase.

7. The method according to claim 5, wherein the ratio of the first salt solution for the salting out of the furostanol saponins in step a is 1:4, initial Tribulus terrestris aqueous extract to salt solution.

8. The method according to anyone of claims 3 to 7, wherein the ratio of the precipitate to salt solution for the washing of the precipitate in step b is 1:1 and step b is repeated five times.

9. The method according to anyone of claims 3 to 7, wherein the volume ratio of the washed precipitate to water in step c is from 1:3, washed precipitate to water.

10. The method according to anyone of claims 3 to 7, wherein the volume ratio of the immiscible in water alcohol in step d is 1:4 water to alcohol.

11. The method according to anyone of claims 3 to 7, wherein the ratio of water for the washing of the alcohol phase in step e is 1:4 water to alcohol phase.

12. The method according to anyone of claims 3 to 11, wherein the initial Tribulus terrestris extract is obtained by a method, comprising the following steps:
a. extracting Tribulus terrestris from ground Tribulus terrestris herb with 20% to 80% lower alcohol in ratio from 1:2 to 1:6 kilogram of herb to liters of alcohol, under stirring conditions, at a temperature of 30°C to 60°C for 2 to 8 hours;
b. repeating the extraction with lower alcohol of step a at least three times and collecting the extracts;
c. concentrating the extracts until the alcohol is completely removed and a first aqueous concentrate is obtained;
d. extracting the Tribulus terrestris from the aqueous concentrate with aqueous butanol in ratio of 1:2 to 1:6 first aqueous concentrate to aqueous butanol, at a temperature of 20°C to 40°C until a butanol phase forms;
e. repeating the extraction with aqueous butanol of step d at least four times and collecting the butanol phases;
f. removing the butanol from the butanol phases until a second aqueous concentrate is obtained;
g. extracting the Tribulus terrestris from the second aqueous concentrate with chloroform by adding chloroform to the second aqueous concentrate in volume ratio from 1:1 to 1:4 second aqueous concentrate to chloroform at a temperature of 20°C to 30°C until aqueous phase forms;
h. repeating the extraction with chloroform of step g at least three times and collecting the aqueous phases;
i. removing the remaining chloroform from the aqueous phases until an aqueous extract of Tribulus terrestris is obtained.

13. The method according to anyone of claims 3 to 11, wherein the final aqueous Tribulus terrestris extract is dried.

14. A Tribulus terrestris extract, obtained by the method of any of the claims 3 to 11, comprising:
i. at least 10% furostanol saponins and
ii. pyrrolizidine alkaloids having concentration of less than 80 µg per liter of said extract.

15. A composition of matter obtained by the method of claim 13, comprising:
i. at least 40% furostanol saponins and
ii. pyrrolizidine alkaloids, having concentration of less than 400 µg per kilogram of said extract.
